Europäisches Patentamt

European Patent Office

Office européen des brevets

(11) Veröffentlichungsnummer: **0 042 818**
**A1**

# (12) EUROPÄISCHE PATENTANMELDUNG

(21) Anmeldenummer: **81810248.5**

(22) Anmeldetag: **17.06.81**

(51) Int. Cl.³: **A 61 K 31/475**
**C 07 D 461/00**

(30) Priorität: **23.06.80 CH 4804/80**

(43) Veröffentlichungstag der Anmeldung:
**30.12.81 Patentblatt 81/52**

(84) Benannte Vertragsstaaten:
**AT BE CH DE FR IT LI LU NL SE**

(71) Anmelder: **CIBA-GEIGY AG**
**Patentabteilung Postfach**
**CH-4002 Basel(CH)**

(72) Erfinder: **Khanna, Satish Chandra, Dr.**
**Rütistrasse 26**
**CH-4103 Bottmingen(CH)**

(54) **Verfahren zur Herstellung von protrahiert wirkenden Vincamin-Zubereitungen, die so erhaltenen Vincamin-Zubereitungen und diese enthaltende Arzneimittel.**

(57) Die vorliegende Erfindung betrifft ein Verfahren zur Herstellung von protrahiert wirkenden und stabilen Vincamin-Zubereitungen, das darin besteht, dass man ein Säureadditionssalz von Vincamin mit einem Alkali- oder Erdalkalimetallsalz eines Sulfonsäure-Kationenaustauscherharzes, insbesondere einem Alkalimetallsalz eines sulfonierten Copolymerisates von Styrol mit wenig Divinylbenzol, in einem polaren Reaktionsmedium, wie Wasser, umsetzt. Weiter betrifft die Erfindung die erfindungsgemäss hergestellten Vincamin-Zubereitungen, deren Verwendung zur Herstellung von gebrauchsfertigen oral verabreichbaren Arzneimitteln und diese selbst.

EP 0 042 818 A1

Croydon Printing Company Ltd.

- 1 -

CIBA-GEIGY AG                 4-12908/=

Basel (Schweiz)

Verfahren zur Herstellung von protrahiert wirkenden Vincamin-Zubereitungen, die so erhaltenen Vincamin-Zubereitungen und diese enthaltende Arzneimittel

Die Erfindung betrifft ein Verfahren zur Herstellung von protrahiert wirkenden Vincamin-Zubereitungen, die so erhaltenen Vincamin-Zubereitungen und diese enthaltende Arzneimittel.

Vincamin und seine pharmazeutisch annehmbaren Säureadditionssalze haben als Arzneistoffe, insbesondere in der Geriatrie, z.B. zur Behandlung von cerebralen Durchblutungsstörungen und cerebraler Hypoxie und deren Folgeerscheinungen, grosses Interesse und bereits auch breite Anwendung in der Praxis gefunden. Die bevorzugte tägliche Dosis von Vincamin oder dessen Hydrochlorid bei oraler Verabreichung beträgt 60 mg, mit Anfangsdosen bis 80 mg und Erhaltungsdosen ab 30 mg; entsprechende Doseneinheitsformen enthalten meist 10, 15, 20 oder insbesondere 30 mg Wirkstoff.

In der publizierten französischen Patentanmeldung 2 253 507, wurde von A. Houdet vorgeschlagen, Vincamin oder dessen Derivate in Arzneiformen mit protrahierter Wirkung zu verabreichen. Als solche Arzneiformen werden insbesondere mit Hilfe von die Wirkstoffabgabe retardierenden Trägerstoffen wie Carboxypolymethylen, Celluloseacetophthalat oder Palmitostearaten von Glycerin hergestellte Tabletten, sowie mit Granulaten der Wirkstoffe gefüllte Gelatinekapseln und, zur intramuskulären Anwendung, Lösungen von Vincamin-Säureadditionssalzen in Polyvinylpyrrolidin genannt und spezifisch beschrieben. Ferner werden auch die Verwendung von Säureadditionssalzen mit verminderter Löslichkeit, wie des bereits in der publizierten französischen Patentanmeldung No. 2.179.538 beschriebenen Pamoats, und schliesslich die

- 2 -

Fixierung von Vincamin an kationischen Ionenaustauschern als Möglichkeiten zur Herstellung von Retardformen der Wirkstoffe, deren protrahierte Wirkung nicht vom verwendeten Trägerstoff abhängig ist, erwähnt,
aber keine weiteren Angaben, z.B. betreffend verwendbare Typen von
Kationaustauschern und die Art der Umsetzung derselben, gemacht. In
der DE-OS 27 07 763 schlägt E.Corvi Mora die Verabreichung von Vincamin
oder dessen Hydrochlorid zur oralen Behandlung von Kreislauferkrankungen des Gehirns in solcher Weise vor, dass zur Erzielung eines
Stoffwechseleffekts eine Konzentration von Vincamin im Blut von 0,1 bis
0,3 µg/ml und zur Erzeugung einer zentralen gefässerweiternden Wirkung
eine solche von 0,2 bis 0,5 µg/ml aufrechterhalten wird. Dies wird
vorzugsweise durch Verabreichung von Vincamin oder dessen Hydrochlorid
in Form eines Arzneimittels mit verzögerter, z.B. sich über 24 Stunden
erstreckender Wirkstofffreisetzung bewirkt. Die verzögerte Wirkstofffreisetzung wird durch Beschichtung der Wirkstoffe mit einer Lipidschicht oder durch Einkapselung in eine geeignete Matrix erreicht.
Eine solche Matrix kann aus Polyäthylenglykolen oder deren Aethern,
Aethylcellulose oder Hydroxypropylcellulose, polymerer Metakieselsäure,
Polyvinylchlorid, Polyvinylacetat, Styrol-Maleinsäure-Kopolymeren und
Naturkautschuk oder deren Mischungen, gegebenenfalls zusammen mit
inerten Füllstoffen bestehen. Die erhaltenen Granulate können in üblicher Weise zur Herstellung von Tabletten, Kapseln, Dragées oder auch
flüssigen Suspensionen verwendet werden.

Protrahiert wirkende Präparate mit Vincamin oder Säureadditonssalzen desselben als Wirkstoffen werden auch schon in erheblicher Zahl
als Handelspräparate in der medizinischen Praxis verwendet, vgl. z.B.
"Rote Liste" 1979, Präparate No. 36066B, 36078B, 36084B, 36085B,
36092B, 36093B und 36095B, sowie als Literatur betreffend die Prüfung
der verzögerten Wirkstoffabgabe aus solchen Präpartent z.B. Pharm.
Ztg. 122, 2067-73 (1977), Dtsch. Apoth.-Ztg. 118, 1-4 (1978),
Therapiewoche 23, 4709-4714 (1978) und Arzneimittel-Forsch. 28, 2332-6
(1978). Die Handelspräparate mit protrahierter Wirkung zur oralen
Verabreichung sind teils Kapseln, teils Tabletten oder Dragées mit
einer Matrix und/oder einem Ueberzug, die die verzögerte Wirkstoffabgabe gewährleisten.

Die Verwendung von Kationenaustauscherharzen zur Herstellung
von pharmazeutischen Zubereitungen mit protrahierter Wirkung aus basischen Wirkstoffen ist an sich schon längere Zeit bekannt. So wird in
der Deutschen Auslegeschrift 1 045 599 die Herstellung von protrahiert
wirkenden Aminzubereitungen durch Umsetzung von therapeutisch wirksamen Aminverbindungen mit Sulfonsäure-Kationenaustauscherharzen beschrieben. Bei letzteren handelt es sich insbesondere um mit 3-12%,
vorzugsweise 5-10% Divinylbenzol vernetzte sulfonierte Polyvinylarylverbindungen, die als freie Säuren, z.B. in Wasser oder Wasser-Methanol
suspendiert, mit Amphetamin, Pyrilamin oder Pyribenzamin als Basen
umgesetzt werden. Gemäss der britischen Patentschrift 857 194 werden
Kationen- oder Anionenaustauscherharze mit jeweils mindestens zwei
basischen bzw. sauren therapeutischen Wirkstoffen zu entsprechenden
Kombinationen vereinigt. Beispielsweise wird die Säureform eines geeigneten, vernetzten Polystyrolsulfonsäureharzes mit der wässrigen Lösung
von einer zur Sättigung nicht ausreichenden Menge Hyoscyamin-sulfat
und anschliessend mit einer wässrigen Lösung von Hyoscin-hydrobromid
umgesetzt.

Abgesehen von der Erwähnung in der obengenannten französischen
Patentanmeldung No. 2,179,538 hat die Bindung von Vincamin an irgendwelche Kationenaustauscherharze keine weitere Beachtung oder gar
praktische Anwendung gefunden. Dies mag damit zusammenhängen, dass die
aus Sulfonsäure-Kationenaustauschharzen und Vincamin direkt gebildeten
Vincamin-Resinate nicht stabil sind. Gemäss eigenen Befunden enthält
bereits die anschliessend an die Umsetzung mit dem Kationenaustauscher
aus diesem durch Feinmahlen, Quellenlassen in wässriger Natriumacetatlösung und Chloroform, anschliessendes Erwärmen des Gemisches auf
60°C unter Rückfluss, Abtrennen und Eindampfen der Chloroformphase
wieder freigesetzte Rohbase gemäss Bestimmung durch Dünnschichtchromatographie oder Liquidchromatographie mindestens 10% Apovincamin und
etwa 5% Epi-Vincamin. Die festgestellte Instabilität führt zur Abnahme
der Wirksamkeit und Veränderung der pharmakologischen Eigenschaften.

- 4 -

Es wurde nun überraschenderweise gefunden, dass man protrahiert
wirkende und stabile Vincamin-Zubereitungen erhält, wenn man ein Säureadditionssalz von Vincamin mit einem Alkali- oder Erdalkalimetallsalz
eines Sulfonsäure-Kationenaustauscherharzes in einem polaren Reaktionsmedium umsetzt.

Als Säureadditionssalze von Vincamin eignen sich sowohl solche
mit anorganischen wie auch solche mit organischen Säuren. Besonders
geeignet sind Additionssalze mit Mineralsäuren, insbesondere mit Halogenwasserstoffsäuren, z.B. der Chlorwasserstoffsäure, sowie mit der
Schwefelsäure, und mit organischen Sulfonsäuren, wie der Methansulfonsäure, aber auch mit Carbonsäuren, wie z.B. der Weinsäure, der Bernsteinsäure oder der Essigsäure.

Grundsätzlich sind alle Sulfonsäure-Kationenaustauscherharze in
Form ihrer Erdalkalimetallsalze und insbesondere ihrer Alkalimetallsalze zur erfindungsgemässen Umsetzung geeignet. Unter Sulfonsäure-
Kationenaustauscherharzen werden neben solchen ohne andere funktionelle
Gruppen als Sulfogruppen auch Phenolsulfonsäure-Kationenaustauscherharze und Carbonsäure-Sulfonsäure-Kationenaustauscherharze wie auch
solche mit weiteren funktionellen Gruppen verstanden. Besonders zufriedenstellende Resultate werden erhalten mit Sulfonierungsprodukten von
Copolymeren von gegebenenfalls indifferent substituierten Styrolen und
geringen Mengen von Divinylbenzolen als Vernetzungsmittel, wie sie in
der US-PS 2 366 007 beschrieben sind. Andere geeignete Kationenaustauscherharze sind z.B. Kondensationsprodukte von Phenolen oder Phenoläthern mit Formaldehyd und gegebenenfalls substituierten Benzolsulfonsäuren, oder Kondensationsprodukte von Formaldehyd mit durch gegebenenfalls veräthertes Hydroxy substituierten aromatischen Sulfonsäuren,
wie die in der US-PS 2 729 607 und der US-PS 2 692 866 als Carbonsäure-
Sulfonsäure-Kationenaustauscherharze beschriebenen Kondensationsprodukte
von Formaldehyd mit Naphthalinsulfonsäuren und Phenoxyessigsäuren bzw.

von Formaldehyd mit den durch Kondensation von Benzaldehyddisulfonsäuren mit zwei Molekülen Phenoxyessigsäure erhaltenen Triphenylmethanderivaten, sowie weitere, in den US-PS 2 204 259 und 2 338 159 beschriebene Kationenaustauscherharze.

Die verwendeten Harze können unvernetzt sein, vorzugsweise verwendet man indessen Harze mit einem Vernetzungsgrad zwischen etwa 1 und
etwa 12%, insbesondere zwischen 4 und 8%. Die Teilchengrösse der verwendeten Harze liegt vorzugsweise zwischen etwa 50 und etwa 1000 µm und
insbesondere, z.B. bei mit Divinylbenzol vernetztem, sulfoniertem Polystyrol, zwischen etwa 100 und etwa 300 µm. Die Wirkstoffabgabegeschwindigkeit nimmt mit zunehmendem Vernetzungsgrad und mit zunehmender
Teilchengrösse ab, deshalb kann eine gewünschte Abgabegeschwindigkeit
bei Harzen mit verschiedener Teilchengrösse durch Aenderung des Vernetzungsgrades eingehalten werden, z.B. durch Verwendung eines Harzes
von vorgenanntem Typ mit einer Teilchengrösse von 150-300 µm und
4%-igem Vernetzungsgrad zur Herstellung von festen oralen Applikationsformen, oder mit einer Teilchengrösse von 50 bis 100 µm und 8%-igem
Vernetzungsgrad zur Herstellung von oral verabreichbaren Suspensionen.

Als polare Reaktionsmedia für die erfindungsgemässe Umsetzung
eignen sich beispielsweise Wasser und polare organische Lösungsmittel,
wie einwertige niedere Alkanole, z.B. Methanol oder Aethanol oder
niedere Alkanone, z.B. Aceton, oder Gemische dieser Lösungsmittel
unter sich und/oder mit Wasser.

Bei der erfindungsgemässen Umsetzung kann, um den Aufwand an
Kationenaustauscherharz und Gesamtgewicht und -volumen der fertigen
Zubereitungen niedrig zu halten, durch entsprechende Wahl der Mengenverhältnisse von Vincaminsäureadditionssalz und Alkali- oder Erdalkalimetallsalz des Kationenaustauschers eine möglichst weitgehende
Sättigung des letzteren angestrebt werden. Der Absättigungsgrad
liegt üblicherweise zwischen etwa 10% und etwa 40%; abgesehen von den Mengenverhältnissen hängt er auch vom verwendeten Harz und von den
Umsetzungsbedingungen ab. Die restlichen Sulfonsäure-anionen bleiben
im wesentlichen durch Alkali- bzw. Erdalkalimetallionen abgesättigt.
Die Umsetzungen erfolgen vorzugsweise unter Rühren bei Raumtemperatur
bzw. 20°C bis etwa 60°C, insbesondere bei etwa 40°C. Sie dauern beispielsweise etwa 5 bis etwa 15 Stunden. Die Reaktionsdauer liegt bei
Verwendung von schwach vernetzten Harzen oder solchen mit kleiner
Partikelgrösse näher bei oder auch unter 5 Stunden und andernfalls
näher bei oder auch über 15 Stunden. Die Aufarbeitung der Umsetzungsprodukte kann in einfacher Weise durch Abfiltrieren und Trocknen,
vorzugsweise bis zur Gewichtskonstanz, z.B. im Vakuum bei Temperaturen
bis etwa 80°C, vorzugsweise bei etwa 50°C, erfolgen.

Die erfindungsgemäss hergestellten Vincamin-Zubereitungen, die
im folgenden auch als Vincamin-Resinate bezeichnet werden, sind im Gegensatz zu den weiter oben erwähnten, durch direkte Umsetzung von Vincamin mit sauren Kationenaustauscherharzen erhaltenen Vincamin-Resinaten
recht stabil; nach 6 Monaten Lagerung eines erfindungsgemäss hergestestellten Resinates von Vincamin mit einem sulfonierten Copolymerisat
von Styrol und Divinylbenzol mit einem Vernetzungsgrad von 4% bei
23°C, bei 35°C und sogar bei 50°C war das in ihm enthaltene Vincamin
noch praktisch unverändert. Ebenfalls nahezu gleichgeblieben war nach
diesen Lagerungsversuchen auch die Wirkstoffabgabegeschwindigkeit.
Die letztere ist auch überraschend wenig abhängig vom pH-Wert der umgebenden Flüssigkeit; bei einem erfindungsgemässen Resinat aus einem
Kationenaustauscherharz vom obengenannten Typ, aber einem Vernetzungsgrad von 8% und einer Partikelgrösse von 150-300 μm entsprach die in
simulierter Magenflüssigkeit vom pH 1,2 innerhalb 4 Stunden abgegebene
Wirkstoffmenge der in simulierter Darmflüssigkeit vom pH 7,5 innerhalb 6 Stunden abgegebenen Wirkstoffmenge. Deshalb ergaben auch Abgabeversuche mit erfindungsgemässen Resinaten aus Harzen vom gleichen
Typ und Vernetzungsgraden von 4%, 6% und 8% mit einer Stunde Verweilzeit in der simulierten Magenflüssigkeit und 7 Stunden in der simulierten Darmflüssigkeit ungebrochen verlaufende Abgabekurven (Abszissen-

- 7 -

achse Zeit, Ordinatenachse freigesetzte Menge Vincamin in % des
Gesamtgehalts).

Die erfindungsgemäss hergestellten Vincamin-Zubereitungen können
als solche oral verabreicht werden, vorzugsweise werden sie aber analog
kristallisierten oder in anderer Weise verfestigten Wirkstoffen zur
Herstellung üblicher Applikationsformen von Arzneimitteln zur oralen
Verabreichung verwendet. Beispielsweise werden sie in üblicher Weise
und mit den üblichen Träger- und Hilfsstoffen zu festen Applikationsformen, z.B. Tabletten, einschliesslich Spezialformen wie Film- oder
Punkttabletten, zu Dragées oder Kapseln, sowie zu flüssigen oralen
Applikationsformen, insbesondere zu Syrups, d.h. aromatisierten
Suspensionen, aber auch zu tropfbaren Suspensionen verarbeitet. Die
Dosierung kann die übliche oder auch leicht vermindert oder erhöht
sein. Dementsprechend kann der Vincamingehalt der einzelnen Doseneinheiten einer halben oder, bei entsprechend verzögerter Wirkstoffabgabe,
die durch übliche retardierende Massnahmen wie Verwendung entsprechender Matrixmassen, Ueberzüge oder Kapseln weiter herabgesetzt sein kann,
auch einer ganzen Tagesdosis entsprechen und deshalb für erwachsene
Menschen von normalem Gewicht insbesondere zwischen 15 bis 30 mg bzw.
30 bis 60 mg liegen, aber auch bis 100 mg betragen.

Die nachfolgenden Beispiele beschreiben die Herstellung von
erfindungsgemässen Vincamin-Resinaten und diese enthaltenden gebrauchsfertigen Applikationsformen, sollen jedoch den Umfang der Erfindung
in keiner Weise beschränken.

Beispiel 1

a) Vorbereitung des Harzes:

1,0 kg Zerolit 225-Na (Markenbezeichnung der Diaprosim Ltd.,
England) sulfoniertes Copolymerisat von Styrol und Divinylbenzol
mit 4% Vernetzung und einer Teilchengrösse von 150-300 µm, Durchschnittsteilchengrösse 225 µm, werden in ungefähr 5 Liter 2-n-Salzsäure suspendiert und die Mischung während 2 Stunden bei 40°C gerührt. Die überstehende Lösung wird dekantiert und das Harz 3 mal mit deionisiertem Wasser gewaschen. Darauf werden 5 Liter 2-n-Natronlauge zugefügt und die Mischung während 6 Stunden bei 40°C gerührt. Das Harz wird mit überschüssigem heissem deionisiertem Wasser gespült bis der pH der abfliessenden Lösung beinahe 5 beträgt. Die angegebenen Verfahrensschritte werden zweimal wiederholt, um eine gute Harzqualität sicher zu stellen. Schliesslich wird das Harz für 2 Stunden in Isopropylalkohol suspendiert, um allfällige organische Verunreinigungen zu entfernen. Das Harz wird darauf filtriert und mit zwei Portionen deionisiertem Wasser gewaschen. Es wird dann bei 50°C im Vakuum bis zur Gewichtskonstanz getrocknet.

b) Beladen des Harzes mit Vincamin:

100 g Vincamin-methansulfonat werden in 1 Liter deionisiertem Wasser gelöst. In dieser Lösung werden 365 g des gemäss obigen Angaben aktivierten Harzes langsam dispergiert. Die Mischung wird dann während 40°C solange gerührt, bis der ganze Aktivstoff ans Harz gebunden ist. Die Restkonzentration des Vincamin-Salzes in der Lösung wird durch ein UV-Spektrosphotometer überwacht. Das erhaltene Vincamin-Resinat wird abfiltriert und bis zur Gewichtskonstanz im Vakuum bei 50°C getrocknet.

c) Herstellung einer oralen Applikationsform:

100 g des erhaltenen getrockneten Vincamin-Resinates werden mit 1 g Magnesiumstearat vermischt und in Kapseln so abgefüllt, dass jede Kapsel einen Vincamin-Gehalt hat, der 100 mg Vincamin-hydrochlorid entspricht.

Beispiel 2

a) Vorbereitung und Beladen des Harzes:

80 g Amberlit IRP 69 M, Teilchengrösse unter 75 $\mu$m (sulfoniertes Copolymeres von Styrol mit ungefär 8% Divinylbenzol, Rohm & Haas Co., Philadelphia), werden vorbereitet, wie im Beispiel 1 beschrieben.

- 9 -

Zum nassen Harz wird eine 50°C warme Lösung zugefügt, die 20 g Vincamin-hydrochlorid in 1 Liter deionisiertem Wasser enthält. Die erhaltene Suspension wird bei 50°C während 24 Stunden gerührt. Das so behandelte Harzmaterial wird abfiltriert und bei 50°C im Vakuum getrocknet. Das getrocknete Resinat enthält Vincamin in einer Menge, die einem Gehalt an 20 Gewichtsprozent Vincamin-hydrochlorid entspricht.

b) Herstellung einer oralen Applikationsform:

4 g Traganth, 1,2 g p-Hydroxybenzoesäuremethylester und 0,3 g p-Hydroxybenzoesäurepropylester werden in einem Liter Wasser bei 80-90°C aufgelöst. Das erhaltene Gel wird abgekühlt und 50 g des oben erhaltenen, trockenen Vincamin-Resinates werden zugefügt und unter Verwendung eines Homogenisators gründlich dispergiert. Dann wird der Dispersion Wasser bis zum Endvolumen von 1 Liter zugefügt, sodass die erhaltene Suspension etwa 5% Vincamin-Resinat enthält. Ein Teelöffel dieser Suspension enthält ungefähr eine Dosis, die 50 mg Vincamin-hydrochlorid entspricht, genau ist diese Dosis in 2,5 ml Suspension enthalten.

Beispiel 3

1,0 kg nach dem von Beispiel 2 hergestelltes, trockenes Vincamin-Resinat wird mit 0,5 kg Lactose und 0,5 kg Maisstärke gemischt und die Mischung mit 0,5 Liter 15%-igem Schleim von Polyvinylpyrroli-don im Kneter geknetet und anschliessend granuliert. Das Granulat wird getrocknet, mit 0,02 kg Aerosil (hochdisperses Silicagel) und 0,02 kg Magnesiumstearat als äussere Phase gemischt und zu Tabletten von 500 mg Gewicht gepresst (Stempeldurchmesser 11,5 mm). Die Tabletten enthalten Vincamin in einer Dosis, die ungefähr 46 mg Vincamin-hydrochlorid entspricht.

Patentansprüche

1. Verfahren zur Herstellung von protrahiert wirkenden und stabilen Vincamin-Zubereitungen, dadurch gekennzeichnet, dass man ein Säureadditionssalz von Vincamin mit einem Alkali- oder Erdalkalimetallsalz eines Sulfonsäure-Kationenaustauscherharzes in einem polaren Reaktionsmedium umsetzt.

2. Verfahren nach Anspruch 1, dadurch gekennzeichnet, dass man ein Alkali- oder Erdalkalimetallsalz eines Sulfonsäure-Kationenaustauscherharzes mit einer Teilchengrösse von etwa 50 bis etwa 1000 µm verwendet.

3. Verfahren nach Anspruch 1, dadurch gekennzeichnet, dass man ein Säureadditionssalz von Vincamin mit einem Alkalimetallsalz eines Sulfonierungsproduktes von Copolymeren von gegebenenfalls substituierten Styrolen mit geringen Mengen von Divinylbenzolen umsetzt.

4. Verfahren nach Anspruch 1, dadurch gekennzeichnet, dass man die Umsetzung in Wasser, in einem niederen Alkanol, einem niederen Alkanon oder Gemischen dieser Lösungsmittel unter sich und/oder mit Wasser durchführt.

5. Verfahren nach Anspruch 1, dadurch gekennzeichnet, dass man als Säureadditionssalz von Vincamin ein Mineralsäuresalz oder ein Salz mit einer organischen Sulfonsäure verwendet.

6. Verfahren nach Anspruch 1, dadurch gekennzeichnet, dass man als Säureadditionssalz von Vincamin Vincamin-methansulfonat oder Vincamin-hydrochlorid verwendet.

7. Protrahiert wirkende und stabile Vincamin-Zubereitung, bestehend aus Vincamin, das mit einem Absättigungsgrad von 10 bis 40% an ein Sulfonsäure-Kationenaustauscherharz gebunden ist, dessen restliche Sulfonsäure-anionen im wesentlichen durch Alkali- oder Erdalkalimetallionen abgesättigt sind.

8.     Vincamin-Zubereitung gemäss Anspruch 7, in der das Vincamin an ein Sulfonsäure-Kationenaustauscherharz mit einer Teilchengrösse von etwa 50 bis etwa 1000 μm gebunden ist.

9.     Vincamin-Zubereitung gemäss Anspruch 7, in der das Vincamin an ein Sulfonierungsprodukt von Copolymeren von gegebenenfalls substituierten Styrolen mit geringen Mengen von Divinylbenzolen gebunden ist.

10.     Vincamin-Zubereitung, hergestellt nach Anspruch 1.

11.     Vincamin-Zubereitung, hergestellt nach Ansprüchen 1 und 2.

12.     Vincamin-Zubereitung, hergestellt nach Ansprüchen 1, 2, 3, 4 und einem der Ansprüche 5 und 6.

13.     Verwendung von Vincamin-Zubereitungen gemäss Anspruch 7, zur Herstellung von Arzneimitteln zur oralen Verabreichung.

14.     Verwendung von Vincamin-Zubereitungen gemäss Anspruch 10 zur Herstellung von Arzneimitteln zur oralen Verabreichung.

15.     Arzneimittel zur oralen Verabreichung, enthaltend eine Vincamin-Zubereitung gemäss Anspruch 7.

16.     Arzneimittel zur oralen Verabreichung, enthaltend eine Vincamin-Zubereitung gemäss Anspruch 10.

Europäisches Patentamt

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| D | <u>FR - A - 2 253 507</u> (A. HOUDET) <br><br> * das ganze Dokument * <br><br> --- | 7 |
| D | <u>DE - B - 1 045 599</u> (E. HAYS et al.) <br><br> * das ganze Dokument * <br><br> --- | 1 |
| | <u>DE - A - 2 707 763</u> (E. CORVI MORA) <br><br> * Patentansprüche * <br><br> --- | 7 |
| | <u>FR - A - 2 201 898</u> (SOGERAS) <br><br> * Patentansprüche * <br><br> --- | 1,7 |
| A | <u>FR - A - 2 313 915</u> (G. CORNEILLE) <br><br> * Patentansprüche * <br><br> --------- | |

**KLASSIFIKATION DER ANMELDUNG (Int Cl.³)**

A 61 K 31/475
C 07 D 461/00

**RECHERCHIERTE SACHGEBIETE (Int. Cl.³)**

C 07 D 461/00
A 61 K 31/395
A 61 K 31/435
A 61 K 31/475

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 22-09-1981 | VAN BIJLEN |

EPA form 1503.1    06.78